# EUROPEAN PATENT APPLICATION

(11) **EP 0 976 826 A1**
(43) Date of publication of application: **02.02.2000**
(21) Application number: 98911083.8
(22) Date of filing: 30.03.1998
(51) Int. Cl.: C12N 15/14, C12N 9/10, C12N 1/15, C12N 1/19, C12N 1/21

(54) **GENE ENCODING RECOMBINANT TREHALOSE PHOSPHORYLASE, VECTOR CONTAINING THE GENE, TRANSFORMANT TRANSFORMED BY THE GENE, AND METHOD FOR PRODUCING RECOMBINANT TREHALOSE PHOSPHORYLASE WITH THE USE OF THE TRANSFORMANT**

(30) Priority: 31.03.1997 JP 9817397
(71) Applicant: Kureha Chemical Industry Co., Ltd., Tokyo 103-8552 (JP)
(72) Inventor: HORINOUCHI, Sueharu, Tokyo 135-0044 (JP); SAITOH, Kohki, Iwaki-shi, Fukushima 974-8232 (JP); TAKAHASHI, Eisaku, Iwaki-shi, Fukushima 974-8232 (JP)
(74) Representative: Forstmeyer, Dietmar, Dr. rer. nat., Dipl.-Chem.
(86) International application number: JP9801423
(87) International publication number: WO9844116

(57) **Abstract**

A novel recombinant trehalose phosphorylase is provided. A gene encoding trehalose phosphorylase has been discovered. A vector containing the gene is constructed, a transformant is prepared, and recombinant trehalose phosphorylase is produced using the transformant. Trehalose phosphorylase can be produced in an industrial scale. Trehalose can be manufactured advantageously in an industry scale from D-glucose and α-D-glucose 1-phosphoric acid using this enzyme.

## Description

### FIELD OF TECHNOLOGY

The present invention relates to a recombinant trehalose phosphorylase, a gene encoding the amino acid sequence of the trehalose phosphorylase, an autonomously replicable vector including the gene, a transformant in which the expression vector of the gene has been introduced, and a method for preparing recombinant trehalose phosphorylase using the same.

### BACKGROUND TECHNOLOGY

Trehalose is a non-reducible disaccharide made by bonding reducing groups in two molecules of glucose. It is widely present in the living world, for example, in bacteria, fungi, algae, plants, insects, animals, and the like. It is known as a reserve substance in organisms or as a substance having a protective function against low temperature and dryness. Because trehalose does not have a reducing group in the molecule, the compound is free from a browning reaction when heated in the presence of an amino acid. It is useful in providing a sweet taste to foods and beverages without causing change of color and without deteriorating the foods and beverages. However, because it has been difficult to obtain a desired amount of trehalose by a conventional method, trehalose has not been used as a sweetener.

Conventional methods for the manufacture of trehalose are broadly classified into (1) a method of utilizing a microorganism, (2) a method of reacting a multienzyme system on carbohydrate, and (3) a method of reacting enzyme on carbohydrate. The first method include, for example, a method of extracting trehalose accumulated in yeast (Japanese Patent Application Laid-open No. 292986/1993), a method of cultivating microorganisms such as those belonging to genus corynebacterium to cause trehalose to be accumulated in the culture broth (Japanese Patent Application Laid-open No. 211882/1993), and the like. Drawbacks of these methods are requirement of impurity removal as an indispensable step, a low yield, and so on. Known as the second method is a method of producing trehalose by reacting a multienzyme system consisting of maltose phosphorylase and β-type trehalose phosphorylase (which means trehalose phosphorylase having β-D-glucose 1-phosphoric acid as a substrate, hereinafter the same) on a substrate which consists of maltose and glucose (Japanese Patent Application Laid-open No. 216695/1983). Using this method trehalose can be separated comparatively easily, but it is difficult to increase the yield of trehalose. This is because the reaction itself is an equilibrium reaction by two types of enzymes, in which the equilibrium is inclining to the production of β-D-glucose 1-phosphoric acid side, the reaction must be carried out using the substrates at a high concentration, which makes it difficult to increase the yield of trehalose. In the third method, maltose is used as a substrate on which an enzyme is reacted and trehalose formed is purified, as described in Japanese Patent Application Laid-open No. 149980/1996. Although a relatively high yield can be achieved by this method, the method requires a several steps of reactions to react potato starch with various enzymes such as α-amylase, isoamylase, β-amylase, glucoamylase, and the like, to obtain maltose. The reaction involves certain problems.

The present inventors have conducted extensive studies about enzymes which can convert trehalose at a high efficiency. As a result, the inventors have found that some kind of microorganisms belonging to genus *Glyfora* reacts on D-glucose and α-D-glucose 1-phosphoric acid and can produce α-type trehalose phosphorylase (which means trehalose phosphorylase having α-D-glucose 1-phosphoric acid as a substrate) which can produce trehalose at a high efficiency. The finding was disclosed in Japanese Patent Application Laid-open No. 255473/1995. Furthermore, the inventors of the present invention have found that sucrose can be converted into trehalose at an efficiency of 90% or more by using sucrose which is a storage sugar of sugar cane and sugar beet and available in a large amount at inexpensive costs as a raw material, and utilizing sucrose phosphorylase and trehalose phosphorylase in a reaction system (Japanese Patent Application Laid-open No 99988/1995). As can be seen in these patent applications, the above-mentioned problems relating to trehalose have been expected to be solved by utilizing α-type trehalose phosphorylase. However, because these microorganisms do not have sufficient enzyme productivity, a great amount of microorganisms must be cultivated to manufacture trehalose in a large scale.

Heretofore, two types of trehalose phosphorylases have been known. One is β-type trehalose phosphorylase having β-D-glucose 1-phosphoric acid as a substrate (Japanese Patent Application Laid-open No. 154485/1975) and the other is α-type trehalose phosphorylase having α-D-glucose 1-phosphoric acid as a substrate (Japanese Patent Application Laid-open No. 255473/1995). However, amino acid sequences or N-terminal amino acids of either of trehalose phosphorylases have not been known at all. For this reason, it is difficult to determine a cDNA sequence of these trehalose phosphorylases. The fact that the microorganisms producing these trehalose phosphorylases are eukaryotic organisms such as green algae and Basidiomycetes increases the difficulty. In view of this situation, to clarify the gene of these trehalose phosphorylases, particularly the base sequence of cDNA, is highly significant in making it easy to supply the enzymes and to improve the physicochemical characteristics, as well as in making the use of the enzymes. Specifically, investigations with important technological and economical significance include improving productivity of these enzymes by introducing cDNA of these trehalose phosphorylases into the same or different microorganisms, utilizing microorganisms having the cDNA of these trehalose phosphorylases as insoluble enzymes, utilizing cDNA of these trehalose phosphorylases or their moieties as a probe in the exploration of a new microorganism which produces trehalose phosphorylases, replacing a part of the base of cDNA of these trehalose phosphorylases to improve their physico-chemical characteristics of these enzymes such as heat stability or pH stability.

### DISCLOSURE OF THE INVENTION

An object of the present invention is to provide a recombinant trehalose phosphorylase which acts on D-glucose and α-D-glucose 1-phosphoric acid to form trehalose by the application of recombinant DNA technology.

Another object of the present invention is to provide a gene encoding the recombinant trehalose phosphorylase.

Still another object of the present invention is to provide a replicable recombination gene comprising the gene.

A further object of the present invention is to provide a transformant into which the recombination gene has been introduced.

A still further object of the present invention is to provide a method for preparing the recombinant trehalose phosphorylase utilizing the transformant.

The present invention has been completed to achieve these objectives.

Specifically, the present invention relates to a novel recombinant trehalose phosphorylase which possesses a function of converting D-glucose and α-D-glucose 1-phosphoric acid into trehalose.

Moreover, the present invention relates to an amino acid sequence of such recombinant trehalose phosphorylase, a gene encoding the amino acid sequence, an autonomously replicable vector comprising the gene, and a transformant prepared by introducing the vector into a host.

The present invention also relates to a method for preparing a recombinant trehalose phosphorylase which comprises cultivating the transformant to cause the recombinant trehalose phosphorylase to express, and collecting the recombinant trehalose phosphorylase from the culture broth.

Specifically, the present invention relates to a gene encoding trehalose phosphorylase which has an amino acid sequence shown by the Sequence ID No. 1 in the sequence table as an N-terminal and an amino acid sequence shown by the Sequence ID No. 2 in the sequence table as a medium fragment sequence.

Furthermore, the gene of the present invention is a gene encoding trehalose phosphorylase which contains the amino acid sequence shown by the Sequence ID No. 3 in the sequence table.

Included in the gene encoding such a trehalose phosphorylase are genome DNA shown by Sequence ID No. 4 in the sequence table and cDNA shown by Sequence ID No. 5 in the sequence table.

The present invention includes such homologous amino acid sequences as those having other amino acids replaced for one or more amino acids, those not having one or more amino acids, or those having additional one or more amino acids, inasmuch as the polypeptides or proteins exhibit trehalose phosphorylase activity. The amino acid sequences which are homologous with such amino acid sequences are also included as far as the polypeptides or proteins exhibit trehalose phosphorylase activity. The present inventors have retrieved polypeptides having a homologous amino acid sequence in the GENETYX-CD protein data base (Ver. 32 NBRF Rel. 46, February, 1996, Software Development Co.), to find polypeptides having homology of INT. Score: 99 or less and 27.6 %/134aa or less. Therefore, amino acid sequences of which at least 50% of the amino acid sequence is identical and which encode trehalose phosphorylase are included in homologous amino acid sequences.

In addition, the present invention includes such homologous base sequences as those having other bases replaced for one or more bases, those not having one or more bases, or those having additional one or more bases according to degeneracy of genetic code, inasmuch as the base sequence encodes a trehalose phosphorylase. In addition, genes which hybridize with the oligonucleotide probe prepared based on the amino acid sequences or base sequences of Sequence ID No. 3 to No. 5 in the sequence table are also included. The hybridization is performed under the conditions of Example 3.

In addition, the amino acid sequence of recombinant trehalose phosphorylase of the present invention has an amino acid sequence shown by Sequence ID No. 1 in the sequence table as a moiety amino acid sequence for the N-terminal, and the amino acids shown by Sequence ID No. 2 in the sequence table as a medium fragment sequence. The amino acid sequence shown by Sequence ID No. 3 of the sequence table is given as such an amino acid sequence.

These amino acid sequences include those having homology with these amino acid sequences. The present invention includes such homologous amino acid sequences as those having other amino acids replaced for one or more amino acids, those not having one or more amino acids, or those having additional one or more amino acids, inasmuch as the polypeptides or proteins exhibit trehalose phosphorylase activity. The recombinant trehalose phosphorylase of the present invention can be prepared by a genetic engineering procedure using the above-mentioned gene.

Moreover, the present invention relates to an autonomously replicable vector which includes the gene encoding the above-mentioned trehalose phosphorylase. Plasmid vector pYES 2.0 can be given as such a vector.

The present invention further relates to a recombinant trehalose phosphorylase expression transformant prepared by introducing such a vector into a host. Bacteria, actinomycetes, yeasts, moulds, and other eukaryotic organisms can be given as such a host.

The present invention also relates to a method for preparing a recombinant trehalose phosphorylase which comprises cultivating the transformant to cause it to express the recombinant trehalose phosphorylase, and collecting the recombinant trehalose phosphorylase from the culture broth.

As a collecting means in the present invention, a conventionally known purification means for collecting enzymes from a culture broth can be used. Given as specific examples of such purification means are centrifugal separation, filtration, concentration, salting-out, dialysis, fractionation sedimentation, ion chromatography, gel filtration chromatography, hydrophobic chromatography, affinity chromatography, electrophoresis, and the like. In the present invention, the recombinant trehalose phosphorylase is collected by using at least one of these means.

The recombinant trehalose phosphorylase of the present invention may be either a purified enzyme or crude enzyme. Moreover, it is possible to use such a transformant as a primary insoluble enzyme, without collecting the enzyme from the recombinant trehalose phosphorylase expression transformant.

### BRIEF DESCRIPTION OF THE DRAWINGS

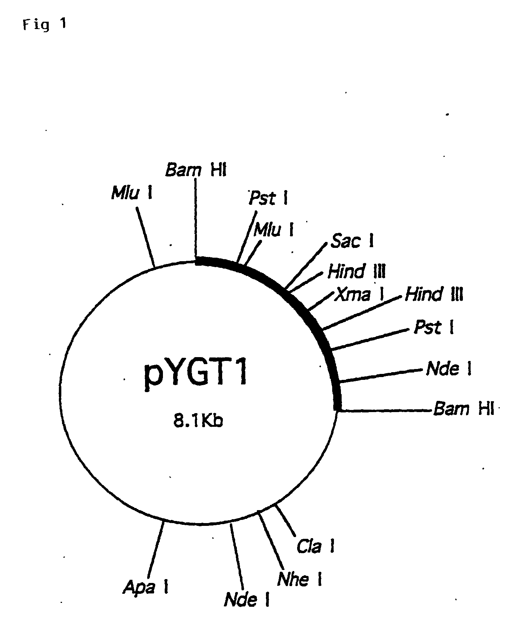

Figure 1 shows a restriction endonuclease map of expression vector pYGRT1 of the present invention.

### THE BEST MODE FOR CARRYING OUT THE INVENTION

The recombinant trehalose phosphorylase of the present invention acts on D-glucose and α-D-glucose 1-phosphoric acid to produce trehalose.

The gene of the present invention can be inserted into an autonomously replicable vector as a replicable recombinant gene. This is introduced into a host which cannot usually produce a recombinant trehalose phosphorylase, but can easily amplify the same to prepare a transformant, which is cultivated to produce a recombinant trehalose phosphorylase.

The transformant of the present invention can produce a recombinant trehalose phosphorylase, if cultivated. A desired amount of recombinant trehalose phosphorylase can be produced by cultivating such a transformant.

To prepare recombinant trehalose phosphorylase, the present inventors have conducted experiments comprising cloning of the trehalose phosphorylase gene produced by *Grifola frondosa* (CM-236, FERM BP No.35) to clarify the base sequence. The details of the experiments will be described below.

### Experimental Example 1

### 〈Amino acid sequence〉

### Experimental Example 1-1

### 〈N-terminal amino acid sequence〉

A protein for sequencing and a peptide (a purified enzyme produced by Example 6 of Japanese Patent Application Laid-open No. 255473/1995) were dissolved in 60 µl of water. Each 20 µl of the solutions was respectively applied to a glass filter disk which previously treated with polybrene. After drying, the sample was set on a primary structure analyzer of protein, PSQ-1 (manufactured by Shimazu Corp.) and sequencing was performed. The solution was loaded to Wakopak WS-PTH (manufactured by Wako Pure Chemicals Co., Ltd.) which had been previously equilibrated with PTH-amino acid eluate, caused to pass through at a flow rate of 1.0 ml/min, and analyzed. Detection was performed at a wavelength of 269 nm. As a result, the purified enzyme was confirmed to possess an amino acid sequence shown by Sequence ID No. 1 in the sequence table on the N-terminal.

### Experimental Example 1-2

### 〈Partial amino acid sequence〉

200 µl of 8 M urea, 50 mM Tris-HCl buffer (pH 9.0) was added to 2 mg of a lyophilized purified enzyme (a purified enzyme produced by Example 6 of Japanese Patent Application Laid-open No. 255473/1995) and incubated for one hour at 37°C. 5 µg of Lysyl endopeptidase and 600 µl of 50 mM Tris-HCl buffer (pH 9.0) were added and incubated for 12 hours at 37°C to partially hydrolyze the enzyme. The hydrolyzate was freeze-dried to terminate the reaction and dissolved in an appropriate amount of 0.1 % (v/v) trifluoroacetic acid. The dissolved hydrolyzate was loaded onto a reverse HPLC column (MCI GEL ODS IMU, manufactured by Mitsubishi Chemical Corp.) which had been equilibrated with 0.1% (v/v) trifluoroacetic acid and fractionated.

Fractions including the peptide fragments which was eluted at about 56 minutes after initiation of elution were collected and dried under vacuum. The product was analyzed in the same manner as in Experimental Example 1-1, to confirm that the peptide fragment possessed the amino acid sequence shown by Sequence ID No. 2 in the sequence table.

Next, a double stranded DNA was prepared using an oligonucleotide chemically synthesized by a DNA synthesizer (a model 381A, manufactured by Applied Biosystems Japan, Ltd.) based on the partial amino acid sequence which was clarified by Experimental Example 1-1 and Experimental Example 1-2, as a probe, and acting a commercially available DNA polymerase (DNA polymerase I, T4 DNA polymerase, etc.) on the genome DNA of *Grifola frondosa*. As a result of extensive retrieval, three DNA fragments which consist of the total of about 8,200 base pairs including the base sequence shown by Sequence ID No. 4 in the sequence table were obtained.

Next, a double stranded cDNA was prepared using an oligonucleotide chemically synthesized based on the signal peptide sequence which was clarified by Example 3 and a poly-A tale as a probe, and acting a commercially available reverse transcriptase (RNA-dependent DNA polymerase, DNA polymerase I, T4 DNA polymerase, etc.) on mRNA of *Grifola frondosa* (CM-236, FERM BP No. 35). As a result of extensive retrieval, a cDNA fragment which consists of the total of about 2,200 base pairs including the base sequence shown by Sequence ID No. 5 in the sequence table was obtained. The base sequence was decoded to confirm that the trehalose phosphorylase produced by the microorganism possesses the amino acid sequence shown by Sequence ID No. 3 in the sequence table which consists of 707 amino acids. In addition, comparison of the base sequences of the cDNA and genome DNA has made it clear that several introns are present.

A series of steps leading to clarification of the amino acid sequences and base sequences shown by Sequence ID Nos. 3, 4, and 5 in the sequence table are summarized as follows.
(1) Trehalose phosphorylase was isolated from the culture broth of donor microorganisms and highly purified to determine the N-terminal amino acid sequence. On the other hand, the purified enzyme was partially hydrolyzed by lysyl endopeptidase, peptide fragments were isolated from the hydrolyzate, and the amino acid sequence was determined.
(2) Liquid nitrogen was added to mycelium of *Grifola frondosa*, the mixture was quickly homogenized, an SDS solution was added to the mixture, and the whole DNA was extracted from the centrifugal supernatant.
(3) A probe of oligonucleotide with a linker which has appropriate restriction endonuclease digesting sites (e.g. those of *Hind* III, *Pst* I, etc.) was prepared based on the amino acid sequence prepared in (1) above. A commercially available DNA polymerase (DNA polymerase I, T4 DNA polymerase, etc. manufactured by Takara Shuzo, etc.) was acted on DNA obtained in (2) above to obtain a DNA which is estimated to be a part of trehalose phosphorylase.
(4) The DNA obtained in (3) above was incorporated into an appropriate vector such as pUC18 plasmid, bacteriophage λ, etc., and transformed by an appropriate microorganism such as *Escherichia coli* JM109 strain. A recombinant DNA was acquired from the transformant by the alkaline method and annealed with a primer. The primer was elongated by acting DNA polymerase thereon. The base sequence of the resulting complemental strand DNA was determined by the Sanger's sequencing method. The amino acid sequence which is estimated from the base sequence was compared with the above-mentioned amino acid sequence on the N-terminal and an amino acid sequence moiety to confirm that the base sequence encodes a trehalose phosphorylase.
(5) A recombinant DNA was extracted from the transformant prepared in (4) above by the alkali method, digested using an appropriate restriction endonuclease such as *Hind* III*, Pst* I*,* etc., subjected to gel electrophoresis to obtain DNA fragments which are moieties of trehalose phosphorylase.
(6) The DNA obtained in (2) above was digested using a suitable restriction endonuclease and subjected to gel electrophoresis. The Southern hybridization was performed using the DNA fragment obtained in (5) as a probe. It was confirmed that DNA fragments including trehalose phosphorylase gene of about 4500 bases can be produced by digestion using restriction endonuclease *Sac* I.
(7) DNA obtained in (2) above was digested using restriction endonuclease *Sac* I and gel electrophoresis was performed. DNA around 4500 bp was extracted and incorporated into an appropriate vector such as pUC18 plasmid, bacteriophage λ, etc. An appropriate microorganism such as *Escherichia coli* JM109 strain was transformed using this vector having the DNA. Selection of colonies including the target DNA was performed by the colony hybridization using DNA fragments obtained in (5) above as a probe, thereby selecting a transformant which includes DNA encoding trehalose phosphorylase.
(8) Recombinant DNA was acquired from the transformant of (7) above, annealed with a primer, and the primer was elongated by the action of DNA polymerase. The resulting complemental strand DNA was analyzed by the Sanger's sequencing method to determine the base sequence. The amino acid sequence estimated from the base sequence was compared with the above-mentioned amino acid sequence on the N-terminal and partial amino acid sequence to confirm that the base sequence encodes a trehalose phosphorylase and that the sequence on the C-terminal is missing.
(9) The recombinant DNA obtained in (8) above was digested using an appropriate restriction endonuclease such as *Sac* I, *Sma* I, etc. and gel electrophoresis was performed to obtain the DNA fragment on the C-terminal side of trehalose phosphorylase.
(10) The DNA obtained in (2) above was digested using a suitable restriction endonuclease and subjected to gel electrophoresis. The Southern hybridization was performed using the DNA fragment obtained in (9) as a probe. It was confirmed that DNA fragments including trehalose phosphorylase gene of about 1700 bases can be produced by digestion using restriction endonuclease *Pst* I.
(11) DNA obtained in (2) above was digested using restriction endonuclease *Pst* I and gel electrophoresis was performed. DNA around 1700 bp was extracted and incorporated into an appropriate vector such as pUC 18 plasmid, bacteriophage λ, etc. An appropriate microorganism such as *Escherichia coli* JML09 strain was transformed using this vector having the DNA. Selection of colonies including the target DNA was performed by the colony hybridization using DNA fragments obtained in (9) above as a probe, thereby selecting a transformant which includes DNA encoding the C-terminal side of trehalose phosphorylase.
(12) Recombinant DNA was acquired from the transformant of (11) above, annealed with a primer, and the primer was elongated by the action of DNA polymerase. The resulting complemental strand DNA was analyzed by the Sanger's sequencing method to determine the base sequence. The base sequence was compared with the base sequence of the above (8) to confirm that the base sequence encodes trehalose phosphorylase. However, since the base sequence was anticipated to be insufficient as the entire base sequence of this enzyme, it was necessary to further acquire the DNA fragment of the C-terminal side.
(13) The recombinant DNA obtained in (12) above was digested using an appropriate restriction endonuclease such as *Sac* I, *Pst* I, etc. and gel electrophoresis was performed to obtain the DNA fragment on the C-terminal side of trehalose phosphorylase.
(14) The DNA obtained in (2) above was digested using a suitable restriction endonuclease and subjected to gel electrophoresis. The Southern hybridization was performed using the DNA fragment obtained in (13) as a probe. It was confirmed that a C-terminal side DNA fragment including trehalose phosphorylase gene of about 3000 bases can be produced by digestion using restriction endonuclease *Sac* I.
(15) DNA obtained in (2) above was digested using restriction endonuclease *Sac* I and gel electrophoresis was performed. DNA around 3000 bp was extracted and incorporated into an appropriate vector such as pUC 18 plasmid, bacteriophage λ, etc. An appropriate microorganism such as *Escherichia coli* JM109 strain was transformed using this vector. Selection of colonies including the target DNA was performed by the colony hybridization using DNA fragments obtained in (13) above as a probe, thereby selecting a transformant which includes DNA encoding the C-terminal side of trehalose phosphorylase.
(16) Recombinant DNA was acquired from the transformant of (15) above, annealed with a primer, and the primer was elongated by the action of DNA polymerase. The resulting complemental strand DNA was analyzed by the Sanger's sequencing method to determine the base sequence. The base sequence was compared with the base sequence of the above (12) to confirm that the base sequence encodes the C-terminal side containing a stop codon of trehalose phosphorylase.
(17) Liquid nitrogen was added to mycelium o*f Grifola frondosa*, the mixture was quickly homogenized, a solution of guanidine thiocyanate and phenol was added to the mixture, and the whole RNA was extracted from the centrifugal supernatant. An mRNA fraction having Poly A was collected using a biotinized oligo(dT) probe.
(18) A double strand cDNA was obtained by acting a specific upstream primer and a specific downstream primer, which have a site responsive to an appropriate restriction endonuclease such as *BamH* I, a commercially available reverse transcriptase, and DNA polymerase (RNA-dependent DNA polymerase, DNA polymerase 1, T4DNA polymerase, etc. manufactured by Takara Shuzo, etc.), on the mRNA produced in (17) above.
(19) The cDNA obtained in (18) above was incorporated into an appropriate vector such as pUC18 plasmid, bacteriophage λ, etc., and transformed using this vector by an appropriate microorganism such as *Escherichia coli* JM109 strain. Selection of colonies including the target cDNA was performed by the colony hybridization using cDNA fragments obtained in (13) above as a probe, thereby selecting a transformant which includes cDNA encoding trehalose phosphorylase.
(20) A recombinant DNA was acquired from the transformant and annealed with a primer. The primer was elongated by acting DNA polymerase thereon. The base sequence of the resulting complemental strand DNA was determined by the Sanger's sequencing method. The amino acid sequence which is estimated from the base sequence was compared with the above-mentioned amino acid sequence on the N-terminal and partial amino acid sequence to confirm that the base sequence encodes a trehalose phosphorylase. It was further found that the DNA of the trehalose phosphorylase possesses several introns.

The trehalose phosphorylase gene of the present invention can be made into a trehalose phosphorylase expression vector by a treatment such as linking of various controlling elements, such as a promoter, which is required for the expression of the gene. Suitable host cells can be transformed using this vector and the transformed host cells can produce trehalose phosphorylase. A mass production technology of trehalose phosphorylase using a genetic engineering procedure is thus established.

Common procedures and methods in the gene recombination technology can be used in the construction of the above-mentioned trehalose phosphorylase expression vector into which the gene of the present invention has been introduced. Included in such procedures and methods are a DNA digestion process using various restriction endonucleases, SI nucleases, etc., a DNA linking process using T4 DNA ligase, etc., agarose gel electrophoresis, a DNA isolation and purification process by polyacrylamide gel electrophoresis, etc., and a DNA recovery and purification process using phenol extraction etc.. Confirmation of a plasmid vector in host cells can be carried out by fractionating the plasmid DNA by an alkali-SDS extraction method (B.C. Birnboim, and J. Doly, Nucletic Acids Res., 7, 1513 (1979)), and the like, processing it by various restriction endonuclease, and verifying presence or absence of the restriction endonucleases, recognizing sites or determining the length of formed DNA fragments. Such confirmation can also be performed by analyzing the base sequence of gene by the Sanger's sequencing method (F. Sanger, et al.., Proc. Natl. Acad. Sci., U.S.A., 74, 5463 (1977)) and the like. Various plasmid vectors such as pUC18 or vectors originating therefrom are suitably used as an original vector for the construction of a recombinant. However, conventionally known various vectors such as various virus vectors, various plasmids, cosmids, and the like, including bacterio phages, animal viruses and plant viruses, can also be used without restriction.

In order to cause the transformant into which the vector possessing the gene of the present invention has been incorporated to express the objective trehalose phosphorylase, such a vector must have, in addition to the gene of the present invention, various controlling elements which are required for the expression such as promoters; transcription elements such as transcription termination signals, poly A chain addition signals (in the case where eucaryotic cells are host cells), etc.; translation elements such as ribosome combining sites; and the like. Given as examples of promoters are promoters for *Escherichia coli* such as *trp* promoter, *lac* promoter, *recA* promoter,*λ PL* promoter, *lpp* promoter, *tac* promoter; promoters for *Bacillus subtilis* such as *SP01* promoter, *SP02* promoter, and *pen* promoter; promoters for yeasts and other eucaryotic cells, such as *pH05* promoter, *GAL1* promoter, *GAL7* promoter, *PGK* promoter, *GAP* promoter, *ADHI* promoter, promoter derived from SV-40; and the like. These controlling elements may be caused to be present in the vector by selecting a plasmid comprising these as an original vector in the construction of the expression vector or by isolating such elements from a plasmid including the same or chemically synthesizing the same for incorporation into a suitable vector. A desired trehalose phosphorylase expression vector can be acquired in this manner. When *Escherichia coli* is used as a host cell, both types of expression vector systems, a system in which the target trehalose phosphorylase is directory expressed in the cells and a system in which the target trehalose phosphorylase is secreted and expressed in periplasm layer, are included.

As the host cell, gram negative bacteria such as *Escherichia coli,* etc., gram positive bacteria such as *Bacillus subtilis,* etc., actinomycetes, yeasts, and animal and plant cells are used without specific limitations. Yeasts are preferred, and among yeasts, YPH 250 (IFO 10502, ATCC96519) of *Saccharomyces cerevisiae* is particularly preferred.

As a method for introducing the expression vector into these host cells and for transforming using the introduced vector, commonly used methods such as a method of processing host cells in an aqueous solution including lithium and adding the vector to this solution (H. Ito, Y. Fukuda, K. Murata, A. Kimura: J. Bacteriol., 153, 163 (1983)) can be given, for example. Such a transformant can be cultivated using a conventional cell culture medium. As examples of the medium which can be used in the cultivation of cells, YPD medium, L-broth medium, E medium, M-9 medium, and the like can be given. Conventionally known various additives such as carbon source substances, nitrogen source substances, inorganic salts, vitamins, natural substance extracts, and physiologically active substances can be added to these media. Cultivation can be carried out by various methods under the conditions such as pH, temperature, aeration, and stirring which are applicable in the growth of host cells. In the case where a yeast is used, this is cultivated under the conditions of a pH of about 5-7.5, and preferably about 6.0-7.0, at a temperature in the range of 16-37°C, preferably 25-35°C, and under stirring by aeration. To increase the amount of expression of the target protein, the above-mentioned medium compositions and culture conditions may be suitably modified according to the objective, such as promotion or suppression of secretion of objective protein.

The trehalose phosphorylase produced and accumulated in this manner can be separated and purified by a conventional method. Using the gene of the present invention, trehalose phosphorylase can be manufactured in a large scale at a high efficiency by a gene recombinant technology. The resulting trehalose phosphorylase can be confirmed by, for example, high performance liquid chromatography (HPLC), polyacrylamide gel electrophoresis (PAGE), and the like. The product can be identified by a conventional structural analysis of polypeptides or proteins such as, for example, molecular weight analysis by SDS-PAGE, isoelectric point measurement by isoelectric-electrophoresis, measurement of amino acid composition by an amino acid analyzer, amino acid sequence determination by an amino acids sequencer, and the like.

Recombinant trehalose phosphorylase can be collected from a culture broth by using at least one enzyme collection means selected from centrifugal separation, filtration, enrichment, salting-out, dialysis, fractionation sedimentation, ion chromatography, gel filtration chromatography, hydrophobic chromatography, affinity chromatography, electrophoresis, and the like. Collected materials may be purified using these means as required.

The present invention is illustrated in more detail by way of embodiments. The procedures shown in these examples are known per se in the art, for example, in "Molecular Cloning - A Laboratory Manual" Second Edition, by J. Samblook, 1989, Cold Spring Harbor Laboratory Publication.

### Example 1

### 〈Preparation of genome DNA〉

*Grifola frondosa* (CM-236, FERM BP No.35) was inoculated into a medium containing 4% of glucose, 0.75% yeast extract, 0.2 % malt extract, 0.5% potassium dihydrogenphosphate, 0.05% magnesium sulfate (pH 5.5), and cultured while shaking at 25°C by a conventional method. 50 ml of the culture broth was separated by centrifugation and washed with water. Cells were collected by suction filtration and crushed in a mortar with the addition of liquid nitrogen. The crushed product was dissolved in 50 mM Tris-HCl buffer (pH 7.6) which contains 100 mM sodium chloride, 5% (w/w) SDS, and 5 mM EDTA, and the mixture was centrifuged. Sodium chloride was added to the supernatant to make a concentration 0.5 M, followed by further centrifugation. 6 ml of 2-propanol was superposed over 10 ml of the supernatant, and the mixture was incubated at room temperature for 30 minutes. Precipitated DNA was transferred to a tube containing 70% ethanol (v/v) using tweezers, washed, and desalted. Furthermore, the DNA was transferred to a tube containing ethanol using tweezers, washed, and desalted. Ethanol was removed by absorbing with a pipet and the DNA was dried under vacuum for one minute. 2 ml of 10mM Tris-HCl buffer (pH 8.0) containing 1 mM EDTA (hereinafter abbreviated as TE) was added to the crude genome DNA thus obtained to dissolve the DNA. After further addition of 2 µg of ribonuclease, the mixture was incubated for 15 minutes at 37°C. 2 ml of chloroform was added to the reaction product, and the mixture was gently stirred and centrifuged. The supernatant was put into a fresh tube, 2 ml of chloroform was added, and the mixture was gently stirred, then centrifuged. The supernatant was transferred to another tube and sodium chloride was added to make a final concentration 0.5 M. 1.2 ml of 2-propanol was superposed over the supernatant, and the mixture was incubated at room temperature for 30 minutes. Precipitated DNA was transferred to a tube containing 70% ethanol (v/v) using tweezers, washed, and desalted. The DNA was further transferred to another tube that contains ethanol using tweezers, washed, and desalted there. Ethanol was removed by absorbing with a pipet and the DNA was dried under vacuum for one minute. 200 µl of TE was added to the purified genome DNA thus obtained. The solution of the genome DNA was lyophilized at -80°C and stored.

### Example 2

### 〈Synthesis of probe DNA〉

Probe 1 of the base sequence, 5'-GGGTACACCAGCCTGCAGCCCATGTGGGC-3' was prepared based on the partial amino acid sequence Gly-Tyr-Thr-Ser-Leu-Thr-Pro-Met-Trp-Ala shown by Sequence ID No. 1. Probe 2 of the base sequence 5'-GGCAAGCTTGATGACCGAGTTCCAAGT-3', which is complementary to Probe 1, was prepared based on the partial amino acid sequence Thr-Trp-Asn-Ser-Val-ILe-Lys shown by Sequence ID No. 2. DNA was amplified by a polymerase chain reaction (PCR) method using these Probes 1 and 2. 1 µl of the genome DNA solution prepared in Example 1 (about 0.2 µg), 1 µl of each probe (about 20 pmol/µl), 0.5 µl of TaKaRa Taq, 10 µl of 10 x PCR Buffer (100 mmM Tris-HCl buffer containing 500 mM potassium chloride and 15 mM magnesium chloride (pH 8.3)), 8 µl of dNTP mixtures (25 mM each), and 78.5 µl of sterile distilled water were added to a 0.2 ml micro-tube. The mixture was reacted using PCR system 2400 (manufactured by Parkin Elmer Co.) at the following temperatures. After the reaction for 5 minutes at 94°C, a 25 cycle reaction was carried out for 1 minute at 95°C, 1 minute at 50°C, and two minutes at 72°C. 5 µl of the resulting reaction product was subjected to agarose electrophoresis to confirm a DNA amplification product of about 800 bp.

The DNA was cut from agarose gel and recovered using Gin Clean Kit (manufactured by Bio 101 Co.) according to the following procedure. An NaI solution 3 times the amount of the separated gel was added and incubated for 5 minutes at 55°C to dissolve the gel. 5 µl of a glass milk was added and blended by overturning. After incubation for 10 minutes over an ice bath, the mixture was centrifuged for 5 seconds at 15,000 x g, the supernatant was removed, and 500 µl of a fresh washing solution was added, followed by blending by overturning and centrifugation for 5 seconds at 15,000 x g again. This procedure was repeated three times. 10 µl of TE was added to the resulting precipitate, the mixture was blended by overturning, and incubated for 15 minutes at 55°C. The mixture was then centrifuged for 30 seconds at 15,000 x g, collecting the resulting supernatant as a DNA solution. Restriction endonuclease*s Hin*d III and *Pst* I, about 10 units each, were added to 5 µl (about 0.1 µg) of the recovered DNA solution, and reacted for one hour to digest the DNA at 37°C, following which 0.1 time an amount of 3M sodium acetate solution (pH 5.2) and 2.5 times an amount of ethanol were added, and the mixture was allowed to stand at -80°C. The mixture was centrifuged to obtain a precipitate. Separately, 0.1 µg of plasmid vector pUC18 (manufactured by Takara Shuzo Co., Ltd.) was digested by restriction endonucleases *Hind* III and *Pst* I according to a conventional method. 0.1 µg of the DNA fragments obtained above and 20 units of T4 DNA ligase were added and the mixture was allowed to stand overnight at 4°C to link the DNA fragments. 100 µl of competent cell JM109 manufactured by Takara Shuzo Co., Ltd. was added to the resulting recombinant DNA, and the mixture was allowed to stand for 30 minutes under cooling with ice, followed by heating to 42°C. 2xYT broth (pH 7.0) was added and the mixture was incubated at 37°C for one hour while shaking, to introduce the recombinant DNA into *Escherichia coli.*

The transformant thus obtained was inoculated into an agar plate medium (pH 7.0) containing 40 µg/ml of 5-bromo-4-chloro-3-indolyl-β-D-galactopiranoside, 10 µg/ml of isopropyl-β-D-thiogalactoside, and 50 µg/ml of ampicillin, and incubated at 37°C for 18 hours to obtain transformed white colonies, which were inoculated into 2 ml of a 2xYT broth (pH 7.0) containing 100 µg/ml of ampicillin. After rotary shaker cultivation at 37°C for 18 hours, cells were collected from the culture broth by centrifugal separation at 15,000 x g for 20 seconds. The supernatant was excluded and 100 µl of Solution 1 (25 mM Tris-HCl buffer containing 50 mM glucose and 10 mM EDTA (pH 8.0)) was added, and the mixture was blended by a boltex mixer and allowed to stand for 5 minutes over an ice bath. 200 µl of Solution 2 (0.2 N sodium hydroxide aqueous solution containing 1% (w/w) of SDS) was added and blended by repeated inversion and the mixture was allowed to stand for 5 minutes over an ice bath.

Next, 150 µl of a solution obtained by blending 60 ml of 5M potassium acetate, 11.5 ml of glacial acetic acid, and 28.5 ml of water was added, and the mixture was blended by overturning and allowed to stand for 10 minutes over an ice bath, followed by centrifugation for 5 minutes at 15,000 x g. The supernatant was recovered and 450 µl of a 1:1 (v/v) phenol-chloroform mixture was added. The mixture was vigorously stirred and centrifuged for 5 minutes at 15,000 x g to recover a supernatant. 1 ml of ethanol was added to the supernatant and the mixture was allowed to stand for 30 minutes in a freezer at -80°C, following which the mixture was again centrifuged for 5 minutes at 15,000 x g to recover a precipitate. The precipitate was washed with 70% (v/v) ethanol, dried in air, dissolved in 50 µl of TE containing 0.5 µg of RNase, and incubated for 30 minutes at 37°C. The incubated solution was added to 30 µl of a 2.5 M sodium chloride solution containing 20% (w/w) of polyethylene glycol 6000, and the mixture was allowed to stand for one hour over an ice bath. The mixture was centrifuged for 10 minutes at 15,000 x g, the precipitate was washed with 70% ethanol, dried in air, and dissolved in 50 µl of TE. 50 µl of a 1:1 (v/v) phenol-chloroform mixture was added to this solution, and the mixture was vigorously stirred, centrifuged for 5 minutes at 15,000 x g to recover a supernatant. 0.1 time the amount of 3M sodium acetate solution (pH 5.2) and 2.5 times the amount of ethanol were added, and the mixture was allowed to stand at -80°C. The mixture was centrifuged to obtain a precipitate.

The precipitate was dissolved in 50 µl of TE and reacted according to the method described in pages 1-4 of "Takara Tag Cycle Sequencing Kit for Shimazu DNA Sequencer Ver. 2 Manual (1995)" published by Takara Shuzo Co., Ltd. Specifically, A, G, C, and T d/ddNTP mixed solutions were added to micro-centrifugal tubes in an amount of 2 µl each. 1 µl of a solution of pUC18 into which DNA fragments have been inserted, 1 µl of M13-forward fluorescence primers or M-13 reverse fluorescence primer, 1.4 of TaKaRa Taq, 5.4 µl of a 5×cycle buffer, and 9.6 µl of sterilizaed water were mixed and charged into the above tubes in an amount of 4 µl each. Each tube was subjected to a reaction using PCR system 2400 (manufactured by Perkin Elmer Co.) at the following temperatures. After the reaction for 3 minutes at 94°C, a 15 cycle reaction was carried out for 30 seconds at 94°C, 30 seconds at 50°C, and one minute at 72°C, followed by a 15 cycle reaction for 30 seconds at 94°C and one minute at 72°C. After the reaction, a termination solution was added 4 µl for each, heat denaturation was performed at 94°C for 5 minutes, and the mixture was quenched. Electrophoresis was performed by preparing a samples using 4% Long-ranger TM gel (AT Biochem) and loading the heat-denatured samples in an amount of 3µl each. In this instance, an edge control solution was loaded simultaneously on both sides of the samples to determine base sequence by Shimazu fluorescence-type sequencer DSQ-1000 (manufactured by Shimazu Corp.). The DNA fragment was judged to be that for trehalose phosphorylase, because the DNA fragment was confirmed to encode the amino acid sequence shown in Sequence ID No. 1 in the sequence table. This DNA has a *Pst* I recognizing site therein and, if a plasmid vector including the DNA is treated with restriction endonucleases *Hind* III and *Pst* I, it is digested into two fragments of about 600 bp and about 200 bp, respectively. The DNA was then processed by a conventional method by the addition of restriction endonucleases *Hind* III and *Pst* I, about 10 units each, and subjected to agarose electrophoresis. DNA amplification products of about 600 bp and about 200 bp were separated from the agarose gel and the resulting DNA fragment obtained by a conventional manner was used as a probe.

### Example 3

### 〈Preparation of trehalose phosphorylase DNA〉

20 units of restriction endonuclease *Sac* I was added to 5 µl (about 1 µg) of the purified genome DNA solution prepared in Example 1, and reacted overnight at room temperature to partially digest genome DNA. After the addition of a 0.1-fold of sodium acetate solution (pH 5.2) and a 2.5-fold of ethanol, the mixture was allowed to stand at -80°C. The mixture was centrifuged to obtain a precipitate. The precipitate thus obtained was dissolved in 10 µl of TE and subjected to agarose gel electophoresis. The agarose gel was immobilized onto a nylon membrane in accordance with the method described in pages 23-28 of "ECL Kit Protocol" (1993), published by Amersham Co. The gel was allowed to stand for 10 minutes in 0.25 N hydrochloric acid solution, washed with water, and shaken for 20 minutes in 0.4 N sodium hydroxide solution. After shaking, DNA was caused to attach to the nylon membrane from the gel by a capillary phenomenon using 0.4 N sodium hydroxide solution. The nylon membrane was washed with a 2 x SSC solution for 5 seconds, dipped in a hybridization buffer containing sodium chloride (0.5M) and blocking agent (5%w/v), and shaken for one hour at 42°C. Separately, labeling was performed in accordance with the method described in pages 11-22 of "ECL Kit Protocol" (1993), published by Amersham Co. About 0.2 µg of the probe DNA prepared in Example 2 was boiled for 5 minutes, quenched over an ice bath, and allowed to stand for 5 minutes. The mixture was centrifuged for 2 seconds at 15,000 x g to cause the solution to sink to the bottom. After the addition of 20 µl of a labeling reagent, and then 20 µl of a glutaraldehyde solution, the mixture was stirred and reacted for 10 minutes at 37°C. The reaction product was stored over an ice bath. The labeled probe was added to a buffer containing the above-mentioned nylon membrane and the mixture was shaken overnight at 42°C. The nylon membrane was removed, immersed into a primary wash buffer (urea 614, SDS 0.4%(w/v), 0.5 x SSC), and shaken for 20 minutes at 42°C. After removing the buffer solution, the nylon membrane was immersed again into the primary wash buffer, and was shaken for 20 minutes at 42°C. The buffer solution was removed, the nylon membrane was immersed into 2 x SSC and shaken for 5 minutes at room temperature. The buffer solution was removed, the nylon membrane was again immersed into 2 x SSC and shaken for 5 minutes at room temperature. The buffer was removed, the nylon membrane was placed on Krewrap (a trademark), a mixture of Reagent 1 and Reagent 2, 2 ml each, was poured onto the nylon membrane, which was then allowed to stand for one minute. The solution was removed, and the genome DNA layer wrapped and immobilized by Krewrap was caused to come contact with an X-ray film, and allowed to stand for 3 minutes. As a result of development, a band indicating a noticeable association was recognized around 4500 bp. Next, 10 µl (about 2 µg) of the genome DNA solution prepared in Example 1 was digested by restriction endonuclease Sac I in accordance with the above-mentioned method, and subjected to agarose gel electophoresis. The DNA around 4500 bp was separated from agarose gel and recovered. Separately, 0.1 µg of plasmid vector pUC18 (manufactured by Takara Shuzo Co., Ltd.) was digested by restriction endonuclease Sac I according to a conventional method. 0.1 µg of the DNA fragments obtained above and 20 units of T4 DNA ligase were added and the mixture was allowed to stand overnight at 4°C to link the DNA fragments. 100 µl of competent cell JM109 manufactured by Takara Shuzo Co., Ltd. was added to the resulting recombinant DNA, and the mixture was allowed to stand for 30 minutes under cooling with ice, followed by heating to 42°C. 2xYT broth (pH 7.0) was added and the mixture was incubated at 37°C for one hour while shaking, to introduce the recombinant DNA into *Escherichia coli.*

The transformant thus obtained was inoculated in an agar plate medium (pH 7.0) containing 40 µg/ml of 5-bromo-4-chloro-3-indolyl-β-D-galactopiranoside, 10 µg/ml of isopropyl-β-D-thiogalactoside, and 50 µg/ml of ampicillin, and incubated at 37°C for 18 hours. About 3,000 white colonies were subjected to colony blotting in accordance with the method described in pages 31-34 of "ECL Kit Protocol" published by Amersham Co. (1993). A nylon membrane with an appropriate size was put on control agar medium, both sides were wetted, and carefully placed on the agar medium in which the colonies were produced. Marks were produced with a sterilized needle on both the nylon membrane and the culture medium so that the direction of colonies can be identified. After one minute, the nylon membrane was removed and placed on a filter paper which had been immersed in a denaturing solution (sodium chloride 1.5 M, sodium hydroxide 0.5 M), with colonies upside, and allowed to stand for 7 minutes. The nylon membrane was placed on a filter paper which contains a neutralization solution (0.5 M Tris-HCl buffer containing 1.5 M sodium chloride and 1 mM EDTA. (pH 7.2)), with the colony side upside, and allowed to stand for 3 minutes. The same procedure was repeated using a fresh filter paper containing the neutralization solution. The nylon membrane was washed with 2 x SSC, placed on a dry filter paper with the colony side upside, and dried in air. Next, the nylon membrane was allowed to stand for 20 minutes on a filter paper in which 0.4 M sodium hydroxide was immersed and washed with 5 x SSC while shaking for 10 to 60 seconds, thereby immobilizing the colonies. Separately, about 0.1 µg of the probe DNA prepared in Example 2 was labeled using an ECL kit (manufactured by Amersham Co.) and hybridized with the colonies of the transformant which were immobilized on the above-mentioned nylon membrane. One transformant exhibiting remarkable association was selected.

This transformant was inoculated in a 2xYT broth (pH 7.0) containing 100 µg/ml of ampicillin, cultivated for 18 hours at 37°C while shaking. After cultivation, the bacterial cells were collected from the culture broth by centrifugation, and recombinant DNA was caused to elute by a conventional alkaline elution method. The product was digested according to a conventional method using various restriction endonucleases (*EcoR* I, *Sac* I, *Kpn* I, *Sma* I, *Bam* HI, *Xba* I, *Sal* I*, Acc* I, *Hinc* II, *Pst* I, *Sph* I, *Hind* III, *Spe* I, *Bln* I, *Fba* I, *Eco* T221, *Bgl* II, *Xho* I), inserted again into plasmid pUC18 and pUC19, purified, and analyzed, to find that the cloned DNA consisted of about 4500 bp. The 4500 bp DNA encodes the DNA of about 2100 bp from the N-terminal of trehalose phosphorylase on the 3'-end. To obtain the DNA on the C-terminal side, 1 µg of the recombinant DNA including 4500 bp was digested by adding restriction endonucleases *Sac* I and *Sma* I, 10 units each, and reacting for 2 hours at 30°C. The digested DNA was subjected to agarose gel electophoresis. DNA around 500 bp digested by *Sac* I and *Sma* I was purified from agarose gel by a conventional method and used as a probe.

About 20 units of restriction endonuclease *Pst* I was added to 5 µl (about 1 µg) of the purified genome DNA solution prepared in Example 1 and reacted overnight at room temperature to digest moiety of the genome DNA. 0.1-fold volume sodium acetate solution (pH 5.2) and 2.5-fold volume ethanol were added to the digested DNA, and the mixture was allowed to stand at -80°C. The mixture was centrifuged to obtain a precipitate. The precipitate thus obtained was dissolved in 10 µl of TE and subjected to agarose gel electophoresis. The agarose gel was immobilized onto a nylon membrane by a conventional method. Separately, about 0.1 µg of probe DNA digested with *Sac* I and *Sma* I was labeled using the ECL kit (manufactured by Amersham Co.) and hybridized with chromosome DNA which was immobilized on the above mentioned nylon membrane, to confirm a band which exhibits remarkable association around 1700 bp. Next, 10 µl (about 2 µg) of the genome DNA solution prepared in Example 1 was digested by restriction endonuclease *Pst* I in accordance with the above-mentioned method, and subjected to agarose gel electophoresis. The DNA around 1700 bp was separated from agarose gel and recovered. Separately, 0.1 µg of plasmid vector pUC18 (manufactured by Takara Shuzo Co., Ltd.) was digested by restriction endonuclease *Pst* I according to a conventional method. 0.1 µg of the DNA fragments obtained above and 20 units of T4 DNA ligase were added and the mixture was allowed to stand overnight at 4°C to link the DNA fragments. 100 µl of competent cell JM109 manufactured by Takara Shuzo Co., Ltd. was added to the resulting recombinant DNA, and the mixture was allowed to stand for 30 minutes under cooling with ice, followed by heating to 42°C. 2xYT broth (pH 7.0) was added and the mixture was incubated at 37°C for one hour while shaking, to introduce the recombinant DNA into *Escherichia coli.*

The transformant thus obtained was inoculated into an agar plate medium (pH 7.0) containing 40 µg/ml of 5-bromo-4-chloro-3-indolyl-β-D-galactopiranoside, 10 µg/ml of isopropyl-β-D-thiogalactoside, and 50 µg/ml of ampicillin, and incubated at 37°C for 18 hours to obtain about 2,000 white colonies, which were immobilized on a nylon membrane. Separately, about 0.1 µg of the probe DNA digested with *Sac* I and *Sma* I was labeled using an ECL kit (manufactured by Amersham Co.) and hybridized with the colonies of the transformant which were immobilized on the above-mentioned nylon membrane. One transformant exhibiting remarkable association was selected.

This transformant was inoculated in a 2xYT broth (pH 7.0) containing 100 µg/ml of ampicillin, cultivated for 18 hours at 37°C while shaking. After cultivation, the bacterial cells were collected from the culture broth by centrifugation, and recombinant DNA was caused to elute by a conventional alkaline elution method. The product was purified and analyzed by a conventional method to confirm that cloned DNA consisted of about 1700 base pairs and did not include the entire base sequence extending to C-terminal.

To obtain the DNA on the C-terminal side, 1 µg of the recombinant DNA including 1700 bp was digested by adding restriction endonucleases *Sac* I and *Pst* I, 10 units each, and reacting for 1 hours at 37°C. The digested DNA was subjected to agarose gel electophoresis. DNA around 500 bp digested by *Sac* I and Pst I was purified from agarose gel by a conventional method and used as a probe. About 20 units of restriction endonuclease *Sac* I was added to 5 µl (about 1 µg) of the purified genome DNA solution prepared in Example 1 and reacted overnight at room temperature to digest a moiety of the genome DNA. 0.1-fold volume sodium acetate solution (pH 5.2) and 2.5-fold volume ethanol were added to the digested DNA, and the mixture was allowed to stand at -80°C. The mixture was centrifuged to obtain a precipitate. The precipitate thus obtained was dissolved in 10 µl of TE and subjected to agarose gel electophoresis. The agarose gel was immobilized onto a nylon membrane by a conventional method. Separately, about 0.1 µg of probe DNA digested with *Sac* I and *Pst* I was labeled using the ECL kit (manufactured by Amersham Co.) and hybridized with genome DNA which was immobilized on the above mentioned nylon membrane, to confirm a band which exhibits remarkable association around 3000 bp. Next, 10 µl (about 2 µg) of the genome DNA solution prepared in Example 1 was digested by restriction endonuclease *Sac* I in accordance with the above-mentioned method, and subjected to agarose gel electophoresis. The DNA around 3000 bp was separated from agarose gel and recovered. Separately, 0.1 µg of plasmid vector pUC18 (manufactured by Takara Shuzo Co., Ltd.) was digested by restriction endonuclease *Sac* I according to a conventional method. 0.1 µg of the DNA fragments obtained above and 20 units of T4 DNA ligase were added and the mixture was allowed to stand overnight at 4°C to link the DNA fragments. 100 µl of competent cell JM109 manufactured by Takara Shuzo Co., Ltd. was added to the resulting recombinant DNA, and the mixture was allowed to stand for 30 minutes under cooling with ice, followed by heating to 42°C. 2xYT broth (pH 7.0) was added and the mixture was incubated at 37°C for one hour while shaking, to introduce the recombinant DNA into *Escherichia coli.*

The transformant thus obtained was inoculated into an agar plate medium (pH 7.0) containing 40 µg/ml of 5-bromo-4-chloro-3-indolyl-β-D-galactopiranoside, 10 µg/ml of isopropyl-β-D-thiogalactoside, and 50 µg/ml of ampicillin, and incubated at 37°C for 18 hours to obtain about 2500 white colonies, which were immobilized on a nylon membrane. Separately, about 0.1 µg of the probe DNA digested with *Sac* I and *Pst* I was labeled using an ECL kit (manufactured by Amersham Co.) and hybridized with the colonies of the transformant which were immobilized on the above-mentioned nylon membrane. One transformant exhibiting remarkable association was selected. This transformant was inoculated in a 2xYT broth (pH 7.0) containing 100 µg/ml of ampicillin, cultivated for 18 hours at 37°C while shaking. After cultivation, the bacterial cells were collected from the culture broth by centrifugation, and recombinant DNA was caused to elute by a conventional alkaline elution method. The product was purified and analyzed by a conventional method to confirm that cloned DNA consisted of about 3000 base pairs and included the base sequence of the C-terminal. It was confirmed that the genome DNA of trehalose phosphorylase consists of about 2750 bp, with a TATA box (RNA polymerase bonding site) at about 50 to 60 bp upstream from the trehalose phosphorylase starting codon ATG, a CT box which is present in yeasts and moulds and functions an important role in the determination of a transcription initiation point at about 10 to 45 bp upstream from the starting codon, and introns which are inserted in several points. Absence of *Bam* HI site was confirmed from the base sequence shown by Sequence ID No. 4 in the sequence table. This was utilized in the following experiments.

### Example 4

### 〈Preparation of trehalose phosphorylase mRNA〉

*Grifola frondosa* was inoculated in a medium comprising glucose (4%), yeast extract (0.75%), malt extract (0.2%), potassium dihydrogenphosphate (0.5%), and magnesium sulfate (0.05%) (pH 5.5), and rotary-shaker-cultured at 25°C for 3 days by a conventional method. 300 ml of the culture broth was separated by centrifugation and washed with sterilized water. Liquid nitrogen was added to 6 g of bacterial cells, which were crushed in a mortar. The crushed product was extracted according to the method described in pages 3-7 of "RNAgent Total RNA Isolation System Technical Bulletin" published by Promega Co. (1993). 4 ml of a denatured solution of guanidine thiocyanate was added to the crushed product and the mixture was stirred for one minute, followed by the addition of 400 µl of 2 M sodium acetate solution (pH 4.0) and 4 ml of a mixed solution consisting of phenol, chloroform, and isoamyl alcohol (25:24:1 (v:v:v)). After blending by overturning, the mixture was stirred for 10 seconds and centrifuged at 15,000 x g for 10 minutes at 4°C to recover 4 ml of a water layer. After further addtion of the 25:24:1 phenol-chloroform-isoamyl alcohol solution, the mixture was stirred for 30 seconds and centrifuged at 15,000 x g for 10 minutes at 4°C to recover 3.8 ml of a water layer. 3.8 ml of isopropanol was added to the water layer and the resulting mixture was allowed to stand for one hour at -20°C. After having been allowed to stand, the mixture was centrifuged at 15,000 x g for one minute at 4°C to recover pellets, which were washed with 70% ethanol, dried, and dissolved in 100 µl of water. About 1 mg of RNA was obtained.

Next, 420 µl of sterilized water was added to 80 µl of the RNA solution. The solution was purified according to the method described in pages 4-7 of "PolyAT tract mRNA Isolation System, Technical Manual" published by Promega Co. (1992). After heating the solution for 10 minutes at 65°C, 3 µl of biotinized oligo(dT) probe (50 pmol/µl) and 13 µl of 20 x SSC were added, the mixture was blended by overturning and allowed to stand for 10 minutes at room temperature. This solution was added to 0.1 ml of 0.5 x SSC which contains Streptavidin MagneSphere Particls (hereinafter called SA-PMPs), blended by overturning, and incubated for 10 minutes at room temperature. After incubation, SA-PMPs was captured by a magnetic stand and the supernatant was removed without disturbing SA-PMPs pellets. 0.3 ml of 0.1 x SSC was added to SA-PMPs for washing. After completely dispersing by overtuning, SA-PMPs was captured by a magnetic stand and the supernatant was removed without disturbing SA-PMPS pellets. This operation was repeated four times. Next, 100 µl of sterilized water was added to SA-PMPs. After completely dispersing by overtuning, SA-PMPs was captured by a magnetic stand and the supernatant was removed without disturbing SA-pMPs pellets. 150 µl of sterilized water was added to repeat the same procedure, thereby obtaining a total of 250 µl of mRNA solution. 25 µl of 3M sodium acetate solution and 275 µl of isopropanol were added to this solution, the mixture was allowed to stand at -20°C overnight, centrifuged at 15,000 x g for 5 minutes at 4°C to recover pellets, The pellets were washed with 70% ethanol, dried, and dissolved in 10 µl of sterilizaed water. 1 µl of the pellets were loaded on an agar plate containing ethidium bromide and ultraviolet ray was irradiated to confirm the presence of mRNA.

### Example 5

### 〈Synthesis of trehalose phosphorylase double stranded cDNA〉

The double stranded cDNA was synthesized in accordance with the method described in pages 2-11 of "RNA LA PCR Kit Manual" (1995), published by Takara Shuzo Co., Ltd. 4 µl of 25 mM magnesium chloride, 2 µl of 10 x RNA PCR buffer, 8 µl of 2.5 mM dNTP mixture, 0.5 µl of RNase inhibitor, 1 µl(50 pmol/µl) of a specific downstream PCR primer (with *a Bam* HI site which is not present in trehalose phosphorylase DNA bonded on a complementary strand of 5'-CGGGGATCCTTTTTTTTTTTTTTTTTT-3'polyA), 1 µl of mRNA purified in Example 4, and 2.5 µl of sterilized water were added to a micro-tube with a volume of 0.2 ml to make the total amount 19 µl. 5 units of reverse transcriptase (1 µl) was added and incubated for 30 minutes at 42°C to synthesize the first strand.

12 µl of 25 mM magnesium chloride, 8 µl of 10 x LA PCR buffer, 1 µl(20 pmol/µl) of a specific upstream PCR primer (with a *Bam* HI site which is not present in trehalose phosphorylase DNA bonded on a probe with a base sequence represented by 5'-CACTGGATCCATGGCTCCTCCCCACCAGTTCCAGTCC-3' of a genome DNA sequence shown by Sequence ID No. 4 in the sequence table 5'-ATGGCTCCTCCCCACCAGTTCCAGTCC-3'), and 58.5 µl of sterilized water were added to the above-mentioned reaction solution to make the total amount 99.5 µl. 2.5 units (0.5 µl) of TaKaRa LA Taq was added and the mixture was incubated for two minutes at 95°C. The following steps 1-3 were repeared 40 cycles to amplify the second strand and cDNA. Step 1: 95°C for 30 seconds; step 2: 67°C for 30 seconds; step 3: 72°C for 3.5 minutes. 20 µl of the reaction solution was subjected to agarose gel electophoresis, to obtain one band around the 2200 bp.

This cDNA around the 2200 bp was separated from agarose gel and recovered, and digested with restriction endonuclease *Bam* HI according to a conventional method. Separately, 0.1 µg of plasmid vector pUC18 (manufactured by Takara Shuzo Co., Ltd.) was digested by restriction endonuclease *Bam* HI according to a conventional method. The cDNA fragments obtained above and 20 units of T4 DNA ligase were added and the mixture was allowed to stand overnight at 4°C to link the DNA fragments. 100 µl of competent cell JM109 manufactured by Takara Shuzo Co., Ltd. was added to the resulting recombinant cDNA, and the mixture was allowed to stand for 30 minutes under cooling with ice, followed by heating to 42°C. 2xYT broth (pH 7.0) was added and the mixture was incubated for one hour at 37°C while shaking, to introduce the recombinant DNA into *Escherichia coli.*

The transformant thus obtained was inoculated into an agar plate medium (pH 7.0) containing 40 µg/ml of 5-bromo-4-chloro-3-indolyl-β-D-galactopiranoside, 10 µg/ml of isopropyl-β-D-thiogalactoside, and 50 µg/ml of ampicillin, and incubated at 37°C for 18 hours to obtain about 20 white colonies, which were immobilized on a nylon membrane. Separately, about 0.1 µg of the probe DNA digested with *Sac* I an*d Pst* I was labeled using an ECL kit (manufactured by Amersham Co.) and hybridized with the colonies of the transformant which were immobilized on the above-mentioned nylon membrane. 18 transformants exhibiting remarkable association were selected.

This transformant was inoculated in a 2xYT broth (pH 7.0) containing 100 µg/ml of ampicillin, cultivated for 18 hours at 37°C while shaking. After cultivation, the bacterial cells were collected from the culture broth by centrifugation, and recombinant DNA was caused to elute by a conventional alkaline elution method. The product was purified and analyzed by a conventional method. The results were as shown by Sequence ID No. 3 and No. 5 in the sequence table. Specifically, a base sequence 1-75 (amino acid sequence 1-25) shows a signal peptide and a base sequence 76-2196 (amino acid sequence 26-732) encodes a matured trehalose phosphorylase polypeptide.

### Example 6

### 〈Construction of expression vector〉

12 units of restriction endonuclease *Bam* HI was added to 1 µg of pUC18 in which the trehalose phosphorylase cDNA obatined in Example 5 had been cloned, and reacted at 30°C for one hour to digest the pUC18. 0.1-fold volume sodium acetate solution (pH 5.2) and 2.5-fold volume ethanol were added and the mixture was allowed to stand at -80°C. The mixture was centrifuged to obtain a precipitate. The precipitate thus obtained was dissolved in 10 µl of TE and subjected to agarose gel electophoresis. A band of about 2200 bp was separated from agarose gel and recovered by a conventional method.

Separately, 12 units of restriction endonuclease *Bam* HI was added to 1 µg of pYES2.0 (manufactured by Invitro Gene Co.), and the mixture was reacted at 30°C for one hour to digest the pYES2.0. 0.1-fold volume sodium acetate solution (pH 5.2) and 2.5-fold volume ethanol were added and the mixture was allowed to stand at -80°C. The mixture was centrifuged to obtain a precipitate, about 0.3 µg of the DNA fragments and 12 units of T4 DNA ligase were added, the mixture was allowed to stand overnight at 4°C, thereby linking the DNA fragments. The resulting recombinant DNA was named "pYGT 1" (Figure 1). 100 µl of competent cell *Saccharomyces cerevisiae* YPH250 (IFO 10502, ATCC 96519) was added and the mixture was allowed to stand for 30 minutes at 30°C. After the addition of 700 µl of TE containing 0.1 M lithium thiocyanate and 30% (w/v) polyethylene glycol 3350, the mixture was incubated for one hour while shaking at 30°C, whereby "pYGT 1" was introduced into yeast.

The transformant thus obtained was inoculated in an agar plate medium which contains 30 µg/ml of lysine, 100 µg/ml of tryptophane, 30 µg/ml of adenine, 30 µg/ml of leucine, and 30 µg/ml of histidine, and incubated for 72 hours at 30°C to select 7 colonies.

### Example 7

### 〈Production of recombinant enzyme by transformant〉

A test tube with a length of 20 cm and an internal diameter of 1.8 cm was charged with 10 ml of a liquid culture medium consisting of 2.5% glucose, 1% yeast extract, 2% bactopeptone, and water, and placed in an autoclave at 121°C for 20 minutes for sterilization. After cooling, the above-mentioned transformant was inoculated in this medium and shake-cultured by a conventional method for 2 days at 30°C. 10 ml of the culture broth was centrifuged to collect cells. Next, a test tube with a length of 20 cm and internal diameter of 1.8 cm was charged with 10 ml of a liquid culture medium consisting pf 3% galactose, 1% yeast extract, 2% bactopepton, and water, and placed in an autoclave at 121°C for 20 minutes for sterilization. After cooling, the collected transformant was suspended again and shake-cultured for 2 days at 30°C by a conventional method. 10 ml of the culture broth was centrifuged to collect cells. The cells were suspended in 300 µl of 40 mM potassium phosphate buffer (pH 7.0) which contains 1 mM glutathione and 1 mM EDTA. About 100 mg of glass beads were added and the cells were crushed by an automatic mixer S-10 (manufactured by Taiyo Kagaku Co., Ltd.). The crushed product was centrifuged at 15,000 x g for 10 minutes, the precipitate was removed to recover the supernatant as a crude enzyme solution.

### Example 8

### 〈Method of measuring activity of crude enzyme solution〉

The following reaction solution was prepared for measuring trehalose phosphorylase activity. 1900 µl of a reaction solution consisting of 40 mM potassium phosphate buffer (pH 7.0), 200 mM trehalose, 10 mM glutathione, 0.16 mM EDTA, 1 mM NADP, 1.3 mM magnesium chloride, 0.067 mM α-glucose-1,6 diphosphoric acid, 1.55 units/ ml phosphoglucomutase (manufactured by Belinger Manheim), 1.75 units/ml glucose-6-phosphoric acid dehydrogenase (manufactured by Belinger Manheim), and sterilized water. 100 µl of of a crude enzyme solution was added to the above reaction solution and reacted at 30°C to measure NADPH produced in one minute at a wavelength of 340 nm. Trehalose is transformed into glucose and α-glucose-1-phosphoric acid by trehalose phosphorylase. The formed α-glucose-1-phosphoric acid is transmuted, together with added α-glucose-1,6 diphosphoric acid, into α-glucose-6-phosphoric acid and α-glucose-1,6 diphosphoric acid by phosphoglucomutase. The formed α-glucose-6-phosphoric acid and added NADP are converted into 6-phosphogluconolactone and NADPH by the action of added glucose-6-phosphoric acid dehydrogenase. As a control, yeast YPH250 having only pYES2.0 which does not include trehalose phosphorylase cDNA was incubated under the same conditions using a liquid culture medium having the same composition. The amount of NADPH produced by the control strain was 1.12x10⁻³ µmol/ml/min. On the other hand, those produced by seven transformed strains were 4.00x10⁻³, 4.61x10^{-3,} 4.29x10⁻³, 3.58x10⁻³, 3.20x10⁻³, 4.81x10⁻³, and 2.80x10⁻³ µmol/ml/min respectively, i.e., about 3.5 times compared to the control showing significantly high values. Also the specific activities of these transformed strains were 4.10x10⁻³, 4.55x10⁻³, 4.34x10⁻³, 3.96x10⁻³, 3.25x10⁻³, 6.44x10⁻³, and 2.74x10⁻³ U/mg-protein (Table 1).

**Table 1**

| Expression of trehalose phosphorylase in yeast | | | | | |
|---|---|---|---|---|---|
| Sample No. | ΔA₃₄₀ | U(Unit) (µmol/ml/ min) | U(-Control) (µmol/ml/min) | Protein conc. (mg/ml) | Specific activity U/mg-protein) |
| Control | 3.47×10⁻⁴ | 1.12×10⁻³ | - | - | - |
| 1 | 1.24×10⁻³ | 4.00×0⁻³ | 2.88×10⁻³ | 7.03×10⁻¹ | 4.10×10⁻³ |
| 2 | 1.43×10⁻³ | 4.61×10⁻³ | 3.49×10⁻³ | 7.67×10⁻¹ | 4.55×10⁻³ |
| 3 | 1.33×10⁻³ | 4.29×10⁻³ | 3.17×10⁻³ | 7.30×10⁻¹ | 4.34×10⁻³ |
| 4 | 1.14×10⁻³ | 3.68×10⁻³ | 2.56×10⁻³ | 6.47×10⁻¹ | 3.96×10⁻³ |
| 5 | 9.93×10⁻⁴ | 3.20×10⁻³ | 2.08×10⁻³ | 6.40×10⁻¹ | 3.25×10⁻³ |
| 6 | 1.49×10⁻³ | 4.81×10⁻³ | 3.69×10⁻³ | 5.73×10⁻¹ | 6.44×10⁻³ |
| 7 | 8.67×10⁻⁴ | 2.80×10⁻³ | 1.68×10⁻³ | 6.13×10⁻¹ | 2.74×10⁻³ |

As described above, a gene of an enzyme which converts D-glucose and α-D-glucose 1-phosphoric acid into trehalose, and trehalose into D-glucose and α-D-glucose 1-phosphoric acid was discovered. This enzyme was produced in the present invention by the application of the recombinant DNA technology.

The recombinant enzyme in the present invention means all enzymes which are prepared by the application of the recombinant DNA technology and which convert D-glucose and α-D-glucose 1-phosphoric acid into trehalose, and trehalose into D-glucose and α-D-glucose 1-phosphoric acid. The recombinant enzyme of the present invention usually possesses the amino acid sequence clarified by the present inventors, for example, the amino acid sequence shown by Sequence ID No. 3 in the sequence table or an amino acid sequences homologous to this. Variants having an amino acid sequence homologous to the amino acid sequence shown by Sequence ID No. 3 can be obtained without changing the target characteristics by replacing one or more amino acids constituting the amino acid sequence of Sequence ID No. 3 with other amino acids, by deleting one or more such constitutional amino acids, or adding one or more other amino acids. In addition, even when using the same DNA, variants lacking one or more amino acids around the N-terminal or C-terminal of the amino acid sequence of the Sequence ID No. 3, or variants added with one or more amino acids around the N-terminal of the amino acid sequence of the Sequence ID No. 3 may be produced according to the host into which the DNA is introduced, components of the nutrition medium used for incubation of transformant including the DNA, culture conditions such as incubation temperature or pH, modification and so on after expression of DNA by host endoenzyme, even if such variants possess the target characteristics. Such variants are also included in the recombinant enzyme of the present invention inasmuch as the variants possess the target characteristics.

The recombinant enzyme of the present invention can be collected from an incubated material of a transformant which includes the specific DNA. The transformant which is used in the present invention can be obtained by introducing DNA with a base sequence, for example, the base sequence which is shown by Sequence ID No. 5 in the sequence table or the base sequence homologous or complementary thereto, into suitable host. In addition, such a base sequence may have one or more bases replaced with other bases without changing an encoding amino acid sequence by utilizing degeneracy of a genetic code. Moreover, it is needless to mention that in order to cause the DNA to express the recombinant enzyme in a host, one or more bases in a base sequence encoding this recombinant enzyme or its homology variant may be suitably replaced with other bases.

The DNA which is used in the present invention may be either a naturally occurring DNA or an artificially synthesized DNA, inasmuch as such a DNA possesses the above-mentioned sequence. As DNA of natural origin, microorganisms belonging to genus *Grifola,* such as *Grifola frondosa* (CM-236, FERM BP No.35) can be given, for example. For example, mRNA which encodes the amino acid sequence shown by Sequence ID No. 3 in the sequence table can be obtained from these microorganisms. Specifically, such microorganisms are inoculated in a nutrition medium, cultivated for a period from one day to one month under aerobic conditions, grown bacterial cells are removed from the culture broth and processed by a cell-wall digesting enzyme such as lysozyme and β-glucanase, crushing, or ultrasonic treatment, whereby mRNA is eluted from the bacterial cells. In this instance, it is possible to use an endopeptidase such as protease in combination with the cell-wall digesting enzyme, to add a surfactant such as SDS when bacterial cells are treated by ultrasonic, or to freeze-thaw the bacterial cells. The target DNA can be obtained by purifying mRNA produced from the product by a conventional method and using a reverse transcriptase, and the like. On the other hand, to artificially synthesize a DNA, the DNA is chemically synthesized based on the base sequence shown by Sequence ID No. 5 in the sequence table, for example, or by a process which comprises inserting the DNA encoding the amino acid sequence shown by Sequence ID No. 3 into a suitable autonomously replicable vector to produce a recombinant DNA, cultivating a transformant which is obtained by introducing the recombinant DNA into a suitable host, collecting bacterial cells from the culture broth, and collecting plasmid including the DNA from the bacterial cells.

DNA is usually introduced into a host in the form of a recombinant DNA. A recombinant DNA usually includes a DNA and an autonomously replicable vector. If a DNA is available, the recombinant DNA can be prepared relatively easily by a common recombinant DNA technology. As examples of such a vector, plasmid vectors such as pBR322, pUC18, Bluescript II SK(+), Pkk223-3, pET 16b, pUB110, pTZ4, pC194, HV14, TRp7, YEp7, pBS7, pYES2.0, and phage vectors such as λ gt · λC, λ gt · λ B, ρ 11, φ 1, φ 105, and the like can be given. Among these pBR322, pUC18, Bluescript II SK(+), pkk223-3, pET16b, λ gt · λ C, and λ gt · λ B are preferable to cause *Escherichia coli* to express the DNA of the present invention, and to pUB110, pTZ4, pC194, ρ 11, φ 1, and φ 105 are preferable to cause *Bacillus subtilis* to express the DNA, and pYES2.0 is preferable to cause yeast to express the DNA. pHV14, TRp7, YEp7, and pBS7 are useful when the recombinant DNA are replicated in two or more types host.

To insert the DNA into such a vector, a common method conventionally used in the art can be employed. Specifically, a gene including DNA and an autonomously replicable vector are digested by a restriction endonuclease or by an ultrasonic treatment, then the produced DNA fragments and vector fragments are linked. If a restriction endonuclease which can specifically act on nucleotide, particularly a type II restriction endonuclease such as *Sau3A* I, *EcoR* I, *Hind* III, *Bam* HI, *Sal* I, *Xba* I, *Sac* I, *Pst* I, or *Bg* III, is used for digesting the gene and vector, DNA fragments and vector fragments can be linked easily. To link DNA fragments and vector fragments, these may be annealed as required, and a DNA ligase may be acted either *in vivo* or *in vitro.* The recombinant DNA thus obtained is introduced into a suitable host, and a transformant thus produced can be infinitely replicated by growing the transformant.

The replicable recombinant DNA thus obtained may be inserted into a suitable host microorganism such as *Escherichia coli*, *Bacillus subtilis*, *actinomycetes*, yeast, and the like. In the case where Escherichia coli is used as a host, the host is grown in the presence of the recombinant DNA and calcium ion; when the host is yeast, on the other hand, a competent cell method, protoplast method, or electroporation method can be applied. When the host is *Bacillus subtilis*, a competent cell method, protoplast method, or electroporation method can be applied. The transformant thus obtained can produce the recombinant enzyme inside and outside the cells, if cultivated in a nutrition medium. As a nutrition medium, a common liquid culture medium containing carbon sources, nitrogen sources, and minerals and, as required, supplemented by trace nutrients such as an amino acid and vitamin. As examples of carbon sources, starch, starch hydrolysate, sugar sources such as glucose, fructose, sucrose, and trehalose can be given. As nitrogen sources, nitrogen-containing organic or inorganic compounds such as ammonia, ammonium salts, urea, nitrates, peptones, yeast extracts, de-fatted soy bean, corn steep liquor, and meat extract can be given for example. The transformant is inoculated in such a nutrition medium, cultivated while maintaining the nutrition medium at a temperature of 20-50°C, pH 2-9, under aerobic conditions by aeration or stirring, for about 1-6 days, to produce a cultured product containing the recombinant enzyme. This culture product can be used as an enzyme agent as is. Usually, however, the enzyme is separated and purified prior to use. The bacterial cells are crushed by a ultrasonic treatment or a boltex mixer, or by using a cytolysis enzyme, and the enzyme is separated from the bacterial cells or crushed bacterial cells by filtration, centrifugation, and the like. For purification, common methods used for purifying enzymes can be used. Specifically, purification means such as condensation, salting-out, dialysis, fractionation, sedimentation, gel filtration chromatography, ion exchange chromatography, hydrophobic chromatography, affinity chromatography, gel electrophoresis, isoelectric electrophoresis, and the like can be used either individually or in combination of two or more.

As mentioned above, the recombinant enzyme of the present invention can convert D-glucose and α-D-glucose 1-phosphoric acid into trehalose, and trehalose into D-glucose and α-D-glucose 1-phosphoric acid. Trehalose possesses a delicately flavored sweet taste. Because trehalose does not possess a reducing group in the molecule, it can be used to provide drinks and beverages with a sweet taste without regard to a risk of discoloration or deterioration. Making use of the above-mentioned characteristics, the recombinant enzyme of the present invention can produce valuable trehalose.

### INDUSTRIAL APPLICABILITY

As described above, the present invention opens the way to efficient and large scale manufacture of an enzyme which can convert D-glucose and α-D-glucose 1-phosphoric acid into trehalose, and trehalose into D-glucose and α-D-glucose 1-phosphoric acid by the application of the recombinant DNA technology. Trehalose possesses a delicately flavored sweet taste. Because trehalose does not possess a reducing group in the molecule, it can be used to provide drinks and beverages with a sweet taste without regard to a risk of discoloration ordeterioration. In addition, because the recombinant enzyme of the present invention is an enzyme of which amino acid sequence has been clarified, the enzyme can be safely used for the manufacture of trehalose to be used as additives to foods, beverages, and pharmaceuticals.

### REMARKS TO DEPOSITED MICROORGANISMS

Name and address of the organization in which the microorganisms have been deposited:
   The Ministry of International Trade and Industry, the Agency of Industrial Science and Technology, Microorganism Technology Institute, 1-1-3, Higashi, Yatabe-cho, Tsukuba-shi, Ibaraki-ken, Japan (Postal Code: 305).
Date of deposition:
   May 1, 1981 (Transferred from FERM No. 3033 of April 22, 1975)
Number of deposition given by the deposition organization:
   FERM BP-35

## Claims

1. A gene encoding trehalose phosphorylase which comprises amino acid sequences shown by Sequence ID No. 1 and Sequence ID No. 2 in the sequence table as partial amino acid sequences or amino acid sequences homologous to these amino acid sequences.

2. A gene encoding trehalose phosphorylase which comprises an amino acid sequence shown by Sequence ID No. 3 in the sequence table or an amino acid sequence homologous to this amino acid sequence.

3. The gene according to claim 1 or 2, comprising the base sequence shown by Sequence ID No. 4 in the sequence table, a base sequence homologous to this base sequence, or a base sequence complementary to this base sequence.

4. The gene according to claim 1 or 2, comprising the base sequence shown by Sequence ID No. 5 in the sequence table, a base sequence homologous to this base sequence, or a base sequence complementary to this base sequence.

5. The gene according to claim 3, which encodes the amino acid sequence shown by Sequence ID No. 3 in the sequence table and in which one or more of the bases in the base sequence shown by Sequence ID No. 4 in the sequence table are replaced by other bases, based on degeneracy of a genetic code.

6. The gene according to claim 4, which encodes the amino acid sequence shown by Sequence ID No. 3 in the sequence table and in which one or more of the bases in the base sequence shown by Sequence ID No. 5 in the sequence table are replaced by other bases, based on degeneracy of a genetic code.

7. The gene according to claim 1 or 2, which hybridizes with an oligonucleotide probe prepared based on the base sequence shown by Sequence ID No. 4 or Sequence ID No. 5 in the sequence table or based on the amino acid sequence shown by Sequence ID No. 3 in the sequence table, under stringent conditions.

8. The gene according to claim 1 or 2, encoding a polypeptide which is identical at least 50% with the polypeptide having the amino acid sequence shown by Sequence ID No. 3 in the sequence table.

9. The gene according to any one of claims 1 to 8, wherein the gene sequence comprises a cDNA sequence, a genome DNA sequence, or a synthetic DNA sequence, originating from mRNA, or a combination of these gene sequences.

10. A recombinant trehalose phosphorylase which comprises an amino acid sequence shown by Sequence ID No. 1 or Sequence ID No. 2 in the sequence table as a partial amino acid sequence or an amino acid sequence homologous to these amino acid sequences.

11. The recombinant trehalose phosphorylase according to claim 10, which comprises the amino acid sequence shown by Sequence ID No. 3 in the sequence table as an amino acid sequence or an amino acid sequence homologous to this amino acid sequence.

12. A recombinant trehalose phosphorylase encoded by the gene of any one of claims 1 to 9.

13. An autonomously replicable vector comprising a gene which encodes the recombinant trehalose phosphorylase encoded according to claim 10 or 11.

14. An autonomously replicable vector comprising a gene which encodes the recombinant trehalose phosphorylase encoded according to claim 12.

15. The autonomously replicable vector according to claim 13 or 14, which is a plasmid vector yYES2.0.

16. A transformant formed by inserting into a host an autonomously replicable vector which comprises a gene encoding the recombinant trehalose phosphorylase according to claim 13.

17. A transformant formed by inserting into a host an autonomously replicable vector which comprises a gene encoding the recombinant trehalose phosphorylase according to claim 14.

18. The transformant according to claim 16 or 17, wherein the autonomously replicable vector is a plasmid vector yYES2.0.

19. The transformant according to claim 16, 17, or 18, wherein the host cell is a bacterium, actinomycete, yeast, mould, or other eukaryotic organism

20. A method for preparing a recombinant trehalose phosphorylase which comprises cultivating the transformant according to any one of claims 16 to 19, to cause the transformant to express the recombinant trehalose phosphorylase in the culture broth, and collecting the recombinant trehalose phosphorylase from the culture broth.

21. The method for preparing a recombinant trehalose phosphorylase according to claim 20, wherein the recombinant trehalose phosphorylase is collected using at least one means selected from the group consisting of centrifugation, filtration, concentration, salting-out, dialysis, fractionation sedimentation, ion chromatography, gel filtration chromatography, hydrophobic chromatography, affinity chromatography, and electrophoresis.
